# EUROPÄISCHE PATENTANMELDUNG

(11) **EP 2 105 897 A2**
(43) Veröffentlichungstag der Anmeldung: **30.09.2009**
(21) Anmeldenummer: 08105773.9
(22) Anmeldetag: 12.11.2008
(51) Int. Cl.: G08B 21/04, A61B 5/00, G06F 19/00

(54) **Verfahren zum Überwachen**

(30) Priorität: 26.03.2008 DE 102008000836
(71) Anmelder: Robert Bosch GmbH, 70442 Stuttgart (DE)
(72) Erfinder: Peters, Oliver, 70469 Stuttgart (DE); Thuersam, Markus, 2300 La Chaux-De-Fonds (CH); Theunissen, Peter, 71717 Beilstein (DE); Maeurer, Christian, 52074 Aachen (DE)

(57) **Zusammenfassung**

Es werden ein Verfahren, eine lokale Verarbeitungseinheit (10) und eine Einrichtung (2) zum Überwachen mindestens einer Person vorgestellt. Bei dem Verfahren zum Überwachen der mindestens einen Person wird die mindestens eine Person durch mindestens einen Sensor (4, 6, 8) erfasst. Von dem mindestens einen Sensor (4, 6, 8) über die mindestens eine Person bereitgestellte Daten (16) werden von der lokalen Verarbeitungseinheit (10) vorverarbeitet. Die vorverarbeiteten Daten (20) werden von der lokalen Verarbeitungseinheit (10) zu einer weiteren Bearbeitung weitergeleitet. Außerdem betrifft die Erfindung ein Computerprogramm und ein Computerprogrammprodukt.

## Beschreibung

Die Erfindung betrifft ein Verfahren zum Überwachen mindestens einer Person, eine lokale Verarbeitungseinheit und eine Einrichtung zur Durchführung des Verfahrens sowie ein Computerprogramm und ein Computerprogrammprodukt.

### Stand der Technik

Sogenannte Assisted Living Systeme werden beispielsweise dazu eingesetzt, ältere und hilfebedürftige Personen aus der Ferne zu überwachen, so dass diese länger in ihrem persönlichen häuslichen Umfeld bleiben können. Diese Systeme bestehen aus verschiedenen Sensoren, bspw. Bewegungsmeldern, Kontaktmatten, Sensoren an Kühlschrank und Toilette. Das Auslösen eines solchen Sensors wird registriert. Mittels einer lokalen Teilnehmerstation werden sämtliche sensorisch ermittelten Daten an eine Empfangszentrale übermittelt. Die Übermittlung erfolgt entweder über das öffentliche Telefonnetz oder eine andere Verbindung zwischen der lokalen Teilnehmerstation und Zentrale.

In der Empfangszentrale werden die Sensordaten typischerweise visualisiert und von einer kompetenten Person bewertet. Zeigen die Daten der Sensoren nach Ansicht der kompetenten Person Auffälligkeiten, werden geeignete Maßnahmen ergriffen, so dass die zu überwachende Person betreut oder in ein Krankenhaus eingewiesen wird.

Weiterhin sind Hausnotrufsysteme bekannt, bei denen ein Signal eines Bewegungsmelders dazu genutzt wird, um einen so genannten Activity monitor zurückzusetzen. Wird dieser Sensor für einen Zeitraum von z. B. 24 h nicht ausgelöst, wird eine Alarmmeldung an die Zentrale übermittelt.

Bei bekannten Systemen führt die Bewertung der gewonnenen Sensordaten oder -informationen durch eine Person dazu, dass der Aufwand für die Betreuung eines einzelnen Patienten erheblich ist. Eine Automatisierung findet derzeit nicht statt. Weiterhin ist vorgesehen, dass die bekannten Hausnotrufsysteme erst nach Ablauf eines langen Zeitraums einen Alarm auslösen. Außerdem können diese nicht als Indikator für eine Verschlechterung des Zustands genutzt werden, sondern wirken nur bei Vorliegen eines echten Notfalls.

Bei sog. Assisted Living Systemen, also Systemen für ein überwachtes und/oder betreutes Wohnen, führt eine Bewertung der Sensordaten durch eine beaufsichtigende Person dazu, dass der Aufwand für die Betreuung eines einzelnen Patienten erheblich ist, eine Automatisierung findet derzeit nicht statt. Bekannte Hausnotrufsysteme lösen erst nach Ablauf eines langen Zeitraums einen Alarm aus und können nicht als Indikator für eine akute Verschlechterung eines Zustands eines Patienten genutzt werden.

Aus der Druckschrift US 5 905 436 ist ein situationsbedingtes Bewachungssystem bekannt, das verschiedene Aktivitäten von Personen in Räumen eines Hauses überwacht und bestimmt, wenn die Person in Schwierigkeiten ist. Hierbei ist vorgesehen, dass die Aktivitäten der Person mit Sensoren erfasst werden. Daten der Sensoren werden an einen zentralen Computer weitergeleitet. Dabei wertet der zentrale Computer sensorisch erfasste Daten nur für Situationen aus, die eine Alarmbedingung erfüllen.

### Offenbarung der Erfindung

Bei dem erfindungsgemäßen Verfahren zum Überwachen mindestens einer Person wird die mindestens eine Person durch mindestens einen Sensor erfasst, wobei von dem mindestens einen Sensor über die mindestens eine Person bereitgestellte Daten von einer lokalen Verarbeitungseinheit vorverarbeitet werden. Die vorverarbeiteten Daten werden von der lokalen Verarbeitungseinheit zu einer weiteren Bearbeitung weitergeleitet.

Im Rahmen des erfindungsgemäßen Verfahrens erfolgt somit eine zweistufige Verarbeitung der sensorisch erfassten Daten. In einem ersten Schritt werden dabei die von dem mindestens einen Sensor erfassten Daten von der lokalen Verarbeitungseinheit vorverarbeitet, was bspw. bedeuten kann, dass durch die lokale Verarbeitungseinheit eine Auswertung und/oder Interpretation der sensorisch erfassten Daten erfolgt. Hierbei können Daten zusammengefasst werden. Es ist ebenfalls möglich, dass auf Grundlage der erfassten Daten von der lokalen Einheit als vorverarbeitete Daten zusätzliche Informationen bereitgestellt werden, die eine zur weiteren Verarbeitung der vorverarbeiteten Daten geeignete Aussage über einen Zustand der mindestens einen Person umfassen.

Bei dem Verfahren können in Ausgestaltung die von dem mindestens einen Sensor erfassten Daten von der lokalen Verarbeitungseinheit ausgewertet werden, wobei auf Grundlage der vorverarbeiteten Daten von der lokalen Verarbeitungseinheit eine Meldung bereitgestellt wird. Weiterhin können in Ausgestaltung die vorverarbeiteten Daten von der lokalen Verarbeitungseinheit zu einer zentralen Station übermittelt und anwenderspezifisch weiterverarbeitet werden.

In einer Variante des Verfahrens ist vorgesehen, dass bei einer anwenderspezifischen Weiterverarbeitung der vorverarbeiteten Daten in der zentralen Station eine beaufsichtigende Person die bereits vorverarbeiteten Daten, die Meldungen und Zusatzinformationen umfassen können, sichtet. Es ist somit nicht mehr erforderlich, dass die beaufsichtigende Person sämtliche von den Sensoren erfasste Daten sichten muss. Da dieser Person die vorverarbeiteten Daten bereitgestellt werden, erhält die Person einen prägnanten Überblick über einen Zustand der mindestens einen zu überwachenden Person, wobei innerhalb der vorverarbeiteten Daten wesentliche Aspekte eines Zustands der mindestens einen zu überwachenden Person hervorgehoben werden können. Somit kann sich die beaufsichtigende Person einen schnellen Überblick über den Zustand verschaffen. Die beaufsichtigende Person ist durch Anwendung des Verfahrens davon entlastet, sämtliche sensorisch erfassten Daten sichten und auswerten zu müssen.

In einer Variante des Verfahrens wird durch die lokale Verarbeitungseinheit ermittelt, ob die von dem mindestens einen Sensor erfassten Daten von einer Norm abweichen. Hierbei können die durch den mindestens einen Sensor erfassten Daten von der lokalen Verarbeitungseinheit mittels eines Algorithmus vorverarbeitet werden. Zum Vorverarbeiten bzw. Auswerten der durch den mindestens einen Sensor erfassten Daten wird von der lokalen Verarbeitungseinheit in einer Variante ein Lernmodus verwendet. Somit ist mit der Erfindung u. a. ein Assisted Living System, d. h. ein System zum betreuten und/oder überwachten Wohnen von mindestens einer zu überwachenden Person umzusetzen. Die lokale Verarbeitungseinheit kann hierbei im Rahmen der Vorverarbeitung mit selbstlernenden Algorithmen ein Verhaltensmuster der mindestens einen zu überwachenden Person dezentral bewerten.

Die erwähnte Norm wird üblicherweise als Kriterium, nach dem die Vorverarbeitung der Daten erfolgt, genutzt. Eine derartige Norm kann üblicherweise vordefiniert werden und durch Normwerte festgelegt sein. Falls beispielsweise sensorisch erfasste Daten außerhalb eines Toleranzbereichs liegen, die im Rahmen der Norm festgelegt sind, können aus den sensorisch erfassten Daten gezielt Informationen bereitgestellt werden, die eine Aussage über den Zustand der mindestens einen zu überwachenden Person liefern. Bei dieser Vorgehensweise kann die Verwendung des Algorithmus innerhalb eines Computerprogramms dienlich sein. Bei der auswertenden Vorverarbeitung der sensorisch erfassten Daten kann durch die lokale Verarbeitungseinheit u. a. untersucht werden, in welchem Maß die sensorisch erfassten Daten von der Norm abweichen. Weiterhin kann auch festgestellt werden, inwiefern die sensorisch erfassten Daten ggf. unabhängig von einer festgelegten Norm bestimmte Regelmäßigkeiten und/oder Unregelmäßigkeiten aufweisen. So kann bspw. erfasst werden, ob die Daten bestimmte Tendenzen aufweisen oder einem Trend unterliegen, was mit Hilfe des Algorithmus festgestellt werden kann.

Mit Hilfe des Lernmodus ist es möglich, ein Schema über eine Verhaltensweise der mindestens einen zu überwachenden Person zu erstellen. Dabei kann festgestellt werden, mit welcher typischerweise zeitabhängigen Regelmäßigkeit die Person bestimmte Tätigkeiten verrichtet. Falls es diesbezüglich Abweichungen geben sollte, also falls die mindestens eine zu überwachende Person eine bestimmte Tätigkeit zu einem ungewohnten Zeitpunkt verrichten sollte, kann dies beim Auswerten und somit Interpretieren der sensorisch erfassten Daten durch die lokale Verarbeitungseinheit berücksichtigt werden, so dass eine geeignete Meldung üblicherweise an die zentrale Station weitergeleitet wird. Die Frage kann durch Bedienen eines bspw. als Tastatur oder Mikrophon ausgebildeten Eingabemodul der Verarbeitungseinheit beantwortet weden.

Weiterhin kann vorgesehen sein, dass die lokale Verarbeitungseinheit von der mindestens einen zu überwachenden Person bedient wird. Dies kann bedeuten, dass die lokale Verarbeitungseinheit aufgrund der vorverarbeiteten Daten der mindestens einen zu überwachenden Person ein Signal übermittelt und ggf. eine Frage stellt. Auf Grundlage einer Beantwortung dieser Frage, wobei zwischen mehreren Antworten ausgewählt werden kann, kann eine entsprechende Meldung durch die lokale Verarbeitungseinheit erzeugt und mit den vorverarbeiteten Daten weitergeleitet werden.

Die Erfindung betrifft weiterhin eine lokale Verarbeitungseinheit zum Überwachen mindestens einer Person, die dazu ausgebildet ist, von mindestens einem Sensor über die mindestens eine Person erfasste Daten vorzuverarbeiten und die vorverarbeiteten Daten zu einer weiteren Bearbeitung weiterzuleiten.

Die erfindungsgemäße Einrichtung zum Überwachen mindestens einer Person weist mindestens einen Sensor, mindestens eine lokale Verarbeitungseinheit und eine zentrale Station auf. Der mindestens eine Sensor ist dazu ausgebildet, Daten über die mindestens eine Person zu erfassen, wobei die lokale Verarbeitungseinrichtung dazu ausgebildet ist, die erfassten Daten vorzuverarbeiten und an die zentrale Einheit weiterzuleiten. Dabei ist die zentrale Einheit dazu ausgebildet, die vorverarbeiteten Daten weiterzuverarbeiten.

Die beschriebene lokale Verarbeitungseinheit oder die Einrichtung ist dazu ausgebildet, sämtliche Schritte des vorgestellten Verfahrens durchzuführen. Dabei können einzelne Schritte dieses Verfahrens auch von einzelnen Komponenten der lokalen Verarbeitungseinheit oder Einrichtung durchgeführt werden. Weiterhin können Funktionen der lokalen Verarbeitungseinheit oder der Einrichtung oder Funktionen von einzelnen Komponenten der Verarbeitungseinheit oder Einrichtung als Schritte des Verfahrens umgesetzt werden.

Die Erfindung betrifft weiterhin einen Computerprogramm mit Programmcodemitteln, um alle Schritte eines beschriebenen Verfahrens durchzuführen, wenn das Computerprogramm auf einem Computer oder einer entsprechenden Recheneinheit, insbesondere in einer vorgestellten lokalen Verarbeitungseinheit oder Einrichtung, ausgeführt wird.

Das erfindungsgemäße Computerprogrammprodukt mit Programmcodemitteln, die auf einem computerlesbaren Datenträger gespeichert sind, ist zum Durchführen aller Schritte eines beschriebenen Verfahrens ausgebildet, wenn das Computerprogramm auf einem Computer oder einer entsprechenden Recheneinheit, insbesondere in einer vorgestellten Einrichtung, ausgeführt wird.

Das erfindungsgemäße Verfahren dient somit zur rechnergestütztenBewachung und sieht vor, dass in einem ersten Schritt durch die lokale Verarbeitungseinheit eine lokale Vorverarbeitung und somit eine erste Verarbeitung von sensorerfassten Daten über bzw. zu der mindestens einen zu überwachenden Person erfolgt. Die vorverarbeiteten Daten werden üblicherweise an die zentrale Station ermittelt und in einem zweiten Schritt weiterverarbeitet. Eine dabei vorgesehene Weiterverarbeitung und somit zweite Verarbeitung der bereits vorverarbeiteten Daten kann anwenderspezifisch von der beaufsichtigenden und/oder betreuenden Person durchgeführt werden.

Die erfindungsgemäße Einrichtung dient zum Überwachen und umfasst mindestens einen Sensor und eine lokale Verarbeitungseinheit, die von den mindestens einem Sensor erfasste Daten vorab verarbeitet, bevor diese an die zentrale Station weitergegeben werden.

Mit der Erfindung ist es in Ausgestaltung möglich, außerordentliche Zustände, wie Notfallsituationen oder ungewöhnliches Verhalten einer Person, bspw. eines Patienten, auf zuverlässigere Weise als bisher bekannt zu erkennen. Dabei ist es nicht nötig, ein das Gesprächs- und Datenaufkommen der zentralen Station, bspw. eines sog. Call-Centers zu erhöhen.

In der zentralen Station, d. h. dem Call-Center, das in einer Variante als Betreuungseinrichtung bezeichnet werden kann, ist in der Regel eine personalintensive Bearbeitung erfasster Daten vorgesehen. Demnach ist der Betrieb der zentralen Station, bedingt durch das dort tätige Personal, bei dem Überwachen von Personen der größte Kostenfaktor.

Im Rahmen der Erfindung werden nunmehr in größerem Umfang Daten erhoben. Diese erhobenen Daten werden bei der lokalen Vorverarbeitung durch die lokale Verarbeitungseinheit auf die relevantesten Daten reduziert. Durch die zentrale Station sind somit statt sämtlicher erhobener Daten nur die vorverarbeiteten Daten im Rahmen des Überwachens zu analysieren oder auszuwerten. Somit kann für den Fall, dass die zentrale Station als ein Call-Center ausgebildet ist, ein anfallendes Gesprächsaufkommen reduziert oder zumindest konstant gehalten werden.

Die erfindungsgemäße Einrichtung bzw. ein entsprechendes System ist derart ausgebildet, dass eine herkömmliche, handelsübliche Vorrichtung bzw. ein herkömmliches, handelsübliches System für betreutes und/oder überwachtes Wohnen (assisted living) durch die lokale Verarbeitungseinheit, die zur lokalen Vorverarbeitung der anfallenden Daten ausgebildet ist, ergänzt wird. Die lokale Verarbeitungseinheit kann durch den mindestens einen Sensor bereitgestellten Daten oder Informationen eine Annahme über den Zustand der Person treffen. Diese lokale Verarbeitungseinheit ist weiterhin dazu geeignet, eine erste Bewertung der Daten zu übernehmen. Nur für den Fall, dass der mindestens eine Sensor unregelmäßige Daten bereitstellt, die bzgl. ihrer Werte bspw. von einer Norm abweichen und/oder ungewöhnliche Werte umfassen, wobei diese definiert werden können, übermittelt die lokale Verarbeitungseinheit eine Information an die zentrale Station.

Die lokale Verarbeitungseinheit empfängt und speichert Informationen die auf Grundlage von Daten, die von dem mindestens einen Sensor, bspw. in einem häuslichen Umfeld durch sensorisch erfasste Ereignisse ausgelöst werden. Die Daten umfassen u. a. zu überwachende personen- oder patientengebundene biometrische Parameter, wie bspw. Frequenz der Atmung oder des Herzschlags, Intensität einer Bewegung der Person, eine Abfolge von Bewegungen der Person, eine Auslösung von Bewegungen und dergleichen.

In einer weiteren Variante der Erfindung, die zudem eine Ausbaustufe der lokalen Verarbeitungseinheit betrifft, kann die lokale Verarbeitungseinheit diese Informationen selbständig erfassen und ggf. lernen, was personengebunden oder -bezogen der Norm entspricht. Somit kann die Norm für biometrische Parameter für eine Person individuell bestimmt werden. Hierbei kann die Norm durch eine Regelmäßigkeit biometrischer Parameter festgelegt werden, was bspw. auf Grundlage von Durchschnittswerten der Parameter erfolgen kann.

Ein hierbei vorgesehenes Lernen kann im einem Fall durch eine Art Lernmodus umgesetzt werden. Hierzu kann die lokale Verarbeitungseinheit für einen festzulegenden Zeitraum in der Größenordnung einiger Tage in diesen Lernmodus geschaltet werden. In einer alternativen Ausgestaltung kann sich die lokale Verarbeitungseinheit fortlaufend in dem Lernmodus befinden. Ein dabei bereitgestelltes Normbild, das in einer Variante zumindest teilweise vordefiniert werden kann, kann ein Vergleichsmaßstab zur Beurteilung dafür dienen, ob eine vorliegende Situation, in der sich die überwachte Person befindet, als normal einzustufen ist oder von der Normalität bzw. Norm abweicht.

Weiterhin kann im Rahmen der Erfindung vorgesehen sein, dass Daten über einen Zustand der Person bzw. des Patienten über einen längeren Zeitraum gespeichert und analysiert werden. Durch eine derartige Maßnahme ist es u. a. möglich, langfristig einen Trend einer Veränderung eines biometrischen Parameters der Person und somit eine möglicherweise vorhandene kontinuierliche Verschlechterung eines Zustands der Person zu überwachen.

In einer ersten Instanz oder einer ersten Stufe hinsichtlich einer Abweichung von der Normalität ist eine Bewertung dieser Daten durch eine Person in Empfangszentrale nicht nötig. Weitere noch effektivere Algorithmen, die durch die lokale Verarbeitungseinheit ausgeführt werden, werten dann die von der Person oder vom Patienten stammenden Daten detaillierter aus und geben ggf. erst danach, in einer zweiten Instanz bzw. einer zweiten Stufe, einen Hinweis an einen Mitarbeiter der zentralen Station.

Die als neue Komponente vorgesehene lokale Verarbeitungseinheit gestattet die lokale Vorverarbeitung der Daten, die von Sensoren u. a. zu biometrischen Parametern bereitgestellt werden. Es können einzelne Informationen von Sensoren mittels lokaler Algorithmen verarbeitet werden. Aus diesen Informationen können auf Grundlage einer zeitlichen Ab- bzw. Reihenfolge von Daten, die von unterschiedlichen Sensoren erfasst werden, ein bestimmtes Ereignis rekonstruiert werden.

In einem Fall können räumlich nebeneinander angeordnete Sensoren durch die zu überwachende Person aktiviert werden. In einem Beispiel wird zunächst der Bewegungsmelder im Schlafraum, eine Trittmatte im Flur bzw. Korridor, eine Bewegungsmelder im Bad, ein durch Betätigung einer Toilettenspülung aktivierter als Schalter ausgebildeter Sensor, dann wieder der Bewegungsmelder im Bad, die Trittmatte sowie der Bewegungsmelder im Schlafraum aktiviert. Falls eine derartige beispielhafte Abfolge einer Aktivierung der verschiedenen Sensoren in einem ggf. als normal zu definierenden Zeitraum, bspw. innerhalb von 5 Minuten erfolgt, wird typischerweise in der lokalen Verarbeitungsstation die Meldung registriert, dass der Toilettenbesuch problemlos war. Demnach wird von der lokalen Verarbeitungseinheit keine Information an die zentrale Station übermittelt. Weiterhin kann die lokale Verarbeitungseinheit Werte für Zeiträume, innerhalb der Toilettenbesuche durchgeführt werden, speichern und somit einen durchschnittlichen Wert für den normalen Zeitraum konkreter bestimmen. Falls der Toilettenbesuch jedoch deutlich länger als der normale Zeitraum andauern sollte, kann die lokale Verarbeitungseinheit als vorverarbeitetes Datum eine entsprechende Meldung registrieren und bedarfsweise eine Information und somit einen Alarm an die zentrale Station weiterleiten, dies kann bspw. dann der Fall sein, wenn der Zeitraum für den Toilettenbesuch mehr als 20 Minuten überschreitet. Alternativ und/oder ergänzend kann eine zeitliche Veränderung von Zeiträumen für Toilettenbesuche und somit allgemein von Werten sensorisch ermittelter Daten und/oder insbesondere biometrischer Parameter registriert und statistisch erfasst werden, falls über einen Zeitraum eine unregelmäßige oder außergewöhnliche Veränderung erfolgt, kann die lokale Verarbeitungseinheit ebenfalls situationsabhängig gezielt eine Meldung und somit Information an die zentrale Station weiterleiten.

Neben der Übertragung einzelner Ereignisse, kann die Einrichtung oder das System, in der Regel die lokale Verarbeitungseinheit, aus der Vielzahl der einzelnen Ereignisse auch einen typischerweise chronologischen Gesamtbefund für die Person erstellen. Führt die Summe der einzelnen Ereignisse zu dem Ergebnis, dass mit der überwachten Person alles in Ordnung ist, so wird als regelmäßig zu erstellender Befund bspw. einmal täglich diese Information an die zentrale Einheit übermittelt.

Die Abläufe und damit die nötigen Algorithmen können für die einzelne Wohnsituation, die Ausstattung mit Sensoren und die mindestens eine Personen, die in dem beobachteten Bereich lebt, individuell bereitgestellt werden. Diese Algorithmen können lokal veränderbar und in der lokalen Verarbeitungseinheit speicherbar sein, um eine ggf. vorzunehmende Individualisierung zu ermöglichen.

Da die Algorithmen teilweise öfters angepasst werden müssen, ist es möglich, eine zwischen der lokalen Verarbeitungseinheit und der zentralen Station vorhandene Datenverbindung auch dazu zu nutzen, um die Algorithmen der lokalen Verarbeitungseinheit aus der Ferne, in der Regel ausgehend von der zentralen Station, zu verändern. In einer weiteren Ausgestaltung kann die lokale Verarbeitungseinheit auch selbstlernend sein.

In die zur Verarbeitung vorgesehenen Algorithmen können auch Informationen eingebunden werden, die durch Eingaben der zu beobachtenden Person ergänzt werden. So kann bspw. sensorisch erfasst werden, dass die Toilette häufiger aufgesucht wird. Diesbezüglich kann von der lokalen Verarbeitungseinheit nachfolgende Meldung an die Person übermittelt werden: "Sie haben innerhalb von 30 min die Toilette siebenmal besucht. Welche Gründe gibt es dafür?". Falls hierzu die in dem Algorithmus eingebundene Rückmeldung: "habe Blumen gegossen" als Antwort der Person bereitgestellt wird, wird registriert, dass alles in Ordnung ist. Falls die Person antworten sollte: "mir geht's nicht gut", kann von der lokalen Einrichtung eine entsprechende Information an die zentrale Einheit weitergeleitet und somit ein Alarm ausgelöst werden.

Weitere Vorteile und Ausgestaltungen der Erfindung ergeben sich aus der Beschreibung und der beiliegenden Zeichnung.

Es versteht sich, dass die voranstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

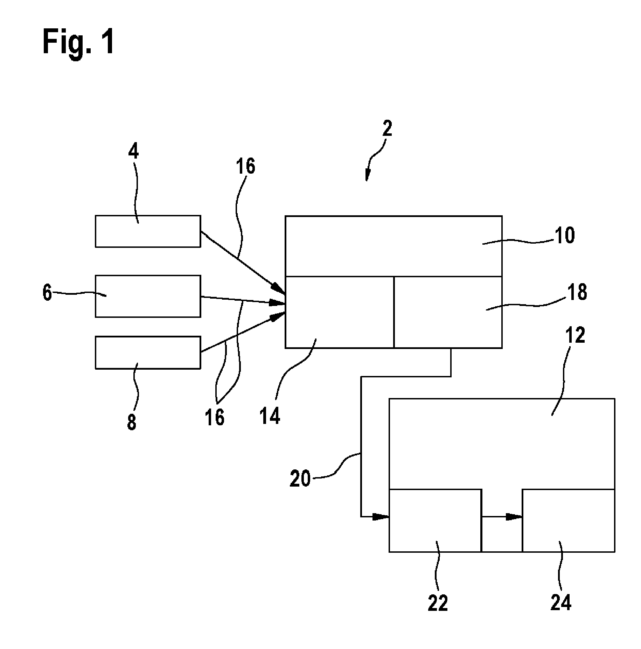

- Figur 1: zeigt eine schematische Darstellung einer Ausführungsform einer erfindungsgemäßen Verarbeitungseinheit als Komponente einer Ausführungsform einer erfindungsgemäßen Einrichtung.

### Kurze Beschreibung der Zeichnungen

Die in Figur 1 schematisch dargestellte Ausführungsform einer erfindungsgemäßen Einrichtung 2 umfasst drei Sensoren 4, 6, 8 und eine als eine häusliche Basisstation ausgebildete Ausführungsform einer erfindungsgemäßen lokalen Verarbeitungseinheit 10. Außerdem umfasst die Einrichtung 2 als eine weitere Komponente eine hier als Call-Center ausgebildete zentrale Station 12.

Im Rahmen einer möglichen Variante des erfindungsgemäßen Verfahrens ist vorgesehen, dass mindestens eine zu überwachende, hier nicht dargestellte Person von den Sensoren 4, 6, 8 erfasst wird. Dies bedeutet in der vorliegenden Variante, dass Bewegungen sowie biometrische Parameter der mindestens einen Person sensorisch überwacht werden. Somit ist mindestens einer der drei Sensoren 4, 6, 8 in Ausgestaltung als ein Bewegungsmelder ausgebildet. Ein weiterer dieser drei Sensoren 4, 6, 8 ist zum Überwachen eines Pulses eines Herzschlags der mindestens einen zu überwachenden Person ausgebildet. Ein dritter Sensore 4, 6, 8 ist durch die Person aufgrund einer Betätigung einer häuslichen Einrichtung mittelbar zu bedienen.

Das beschriebene Verfahren wird in der hier beschriebenen Variante zweistufig ausgeführt. Ein Vorverarbeitungsmodul 14, das als eine Komponente der lokalen Verarbeitungseinheit 10 ausgebildet ist, ist zum Vorverarbeiten von Daten 16, die von den Sensoren 4, 6, 8 beim Überwachen der mindestens einen Person bereitgestellt werden, ausgebildet. Bei einer Vorverarbeitung, die durch das Vorverarbeitungsmodul 14 ausgeführt wird, werden diese sensorisch erfassten Daten 16 ausgewertet und interpretiert, wobei in der beschriebenen Variante ein zusammenfassender Überblick über die Daten 16 bereitgestellt wird.

Zum Vorverarbeiten der sensorisch erfassten Daten 16 durch das Vorverarbeitungsmodul 14, wird durch das Vorverarbeitungsmodul 14 und somit von der lokalen Verarbeitungseinheit 10 ein erster Teil einer Algorithmik verwendet.

Die lokale Verarbeitungseinheit 10 weist als weitere Komponente ein Übertragungsmodul 18 auf, das dazu ausgebildet ist, auf Grundlage der sensorisch erfassten Daten 16 die von dem Vorverarbeitungsmodul bereitgestellten vorverarbeiteten Daten 20 an die zentrale Einheit 12 weiterzuleiten. Diese vorverarbeiteten Daten 20 werden von einem Weiterverarbeitungsmodul 22, das als eine Komponente der zentralen Station 12 ausgebildet ist, weiter verarbeitet.

Beim Weiterverarbeiten der vorverarbeiteten Daten 22 durch das Weiterverarbeitungsmodul 22 und somit durch die zentrale Station 12 wird ein zweiter Teil einer Algorithmik verwendet.

Die zentrale Station 12 kann weiterhin ein Anzeigemodul 24 aufweisen, mit dem die weiterverarbeiteten Daten, die aus den vorverarbeiteten Daten 20 bereitgestellt werden, einer Person, die zum Beaufsichtigen der mindestens einen zu überwachenden Person vorgesehen ist, weitergeleitet werden.

## Patentansprüche

1. Verfahren zum Überwachen mindestens einer Person, bei dem die mindestens eine Person durch mindestens einen Sensor (4, 6, 8) erfasst wird, wobei von dem mindestens einen Sensor (4, 6, 8) über die mindestens eine Person bereitgestellte Daten (16) von einer lokalen Verarbeitungseinheit (10) vorverarbeitet werden, und wobei die vorverarbeiteten Daten (20) von der lokalen Verarbeitungseinheit (10) zu einer weiteren Bearbeitung weitergeleitet werden.

2. Verfahren nach Anspruch 1, bei dem die von dem mindestens einen Sensor (4, 6, 8) erfassten Daten (16) von der lokalen Verarbeitungseinheit (10) ausgewertet werden, wobei auf Grundlage der vorverarbeiteten Daten (20) von der lokalen Verarbeitungseinheit (10) eine Meldung bereitgestellt wird.

3. Verfahren nach Anspruch 1 oder 2, bei dem die vorverarbeiteten Daten (20) von der lokalen Verarbeitungseinheit (10) zu einer zentralen Station (12) übermittelt und anwenderspezifisch weiterverarbeitet werden.

4. Verfahren nach einem der voranstehenden Ansprüche, bei dem durch die lokale Verarbeitungseinheit (10) ermittelt wird, ob die von dem mindestens einen Sensor (4, 6, 8) erfassten Daten (16) von einer Norm abweichen.

5. Verfahren nach einem der voranstehenden Ansprüche, bei dem die durch den mindestens einen Sensor (4, 6, 8) erfassten Daten (16) von der lokalen Verarbeitungseinheit (10) mittels eines Algorithmus vorverarbeitet werden.

6. Verfahren nach einem der voranstehenden Ansprüche, bei dem die durch den mindestens einen Sensor (4, 6, 8) erfassten Daten (16) von der lokalen Verarbeitungseinheit (10) mittels eines Lernmodus vorverarbeitet werden.

7. Verfahren nach einem der voranstehenden Ansprüche, bei dem die lokale Verarbeitungseinheit (10) von der mindestens einen zu überwachenden Person bedient wird.

8. Lokale Verarbeitungseinheit zum Überwachen mindestens einer Person, die dazu ausgebildet ist, von mindestens einem Sensor (4, 6, 8) über die mindestens eine Person erfasste Daten (16) vorzuverarbeiten und die vorverarbeiteten Daten (20) zu einer weiteren Bearbeitung weiterzuleiten.

9. Einrichtung zum Überwachen mindestens einer Person, die mindestens einen Sensor (4, 6, 8), mindestens eine lokale Verarbeitungseinheit (10) und eine zentrale Station (12) aufweist, bei der der mindestens eine Sensor (4, 6, 8) dazu ausgebildet ist, Daten (16) über die mindestens eine Person zu erfassen, wobei die lokale Verarbeitungseinrichtung (10) dazu ausgebildet ist, die erfassten Daten (16) vorzuverarbeiten und an die zentrale Einheit (12) weiterzuleiten, und wobei die zentrale Einheit (12) dazu ausgebildet ist, die vorverarbeiteten Daten (20) weiterzuverarbeiten.

10. Computerprogramm mit Programmcodemitteln, um alle Schritte eines Verfahrens nach einem der Ansprüche 1 bis 7 durchzuführen, wenn das Computerprogramm auf einem Computer oder einer entsprechenden Recheneinheit, insbesondere in einer lokalen Verarbeitungseinheit (10) nach Anspruch 8, ausgeführt wird.

11. Computerprogrammprodukt mit Programmcodemitteln, die auf einem computerlesbaren Datenträger gespeichert sind, um alle Schritte eines Verfahrens nach einem der Ansprüche 1 bis 7 durchzuführen, wenn das Computerprogramm auf einem Computer oder einer entsprechenden Recheneinheit, insbesondere in einer lokalen Verarbeitungseinheit (10) nach Anspruch 8, ausgeführt wird.
